# EUROPEAN PATENT SPECIFICATION

(11) **EP 1 683 516 B1**
(45) Date of publication and mention of the grant of the patent: **02.06.2010**
(21) Application number: 06250301.6
(22) Date of filing: 20.01.2006
(51) Int. Cl.: A61K 9/14, A61K 9/16, A61K 31/423

(54) **Stable pharmaceutical formulations of zonisamide and methods for their manufacture**
Stabile pharmazeutische Zusammensetzungen von Zonisamide und Verfahren zur deren Herstellung
Compositions pharmaceutiques stables de zonisamide et leur procédé de fabrication

(30) Priority: 21.01.2005 US 645030 P
(43) Date of publication of application: 26.07.2006
(60) Divisional application: 07020984.6
(73) Proprietor: Teva Pharmaceutical Industries Ltd, 49131 Petah Tiqva (IL)
(72) Inventor: Hrakovsky, Julia, Rosh Ha-Ayin 48057 (IL); Tenengauzer, Ruth, Hod Hasharon 45322 (IL)
(74) Representative: Gallagher, Kirk James

(56) References cited:
- EP-A- 0 409 254
- EP-A- 1 040 830
- WO-A-02/31499
- US-A1- 2004 047 908
- US-A1- 2004 138 474
- DATABASE WPI Section Ch, Week 198831 Derwent Publications Ltd., London, GB; Class A96, AN 1988-215492 XP002377546 & JP 63 150220 A (DAINIPPON PHARM CO LTD) 22 June 1988 (1988-06-22) -& DATABASE CA [Online] CHEMICAL ABSTRACTS SERVICE, COLUMBUS, OHIO, US; NAKAMURA, YASUHIKO ET AL: "Control of unpleasant tastes of pharmaceuticals" XP002377800 retrieved from STN Database accession no. 1989:141574

## Description

### FIELD OF THE INVENTION

The present invention relates to the stable pharmaceutical dosage form of claim 1 comprising zonisamide as the active ingredient . The invention also concerns the process of claim 10 and the composition of claim 21.

### BACKGROUND OF THE INVENTION

Zonisamide is known as 1,2-benzisoxazole-3-methane sulfonamide or 3-(sulfamoylmethyl)-1,2-benzisoxazole. It is currently available, in capsules, as an anti-epileptic agent which possesses anti-convulsant and anti-neurotoxic effects.

WO 02/31492 discloses a capsule comprising zonisamide.

Zonisamide capsules in the prior art can be prepared by a conventional process of mixing powders of zonisamide and suitable pharmaceutical excipients followed by filling hard gelatin capsule shells with the mixed powders to form the capsules. However, zonisamide capsules prepared by the prior art process suffer at least two disadvantages. First, the powders inside the prior art zonisamide capsules become lumpy upon storage of the capsules. Second, as a result of the lumpy powders, the release rate of zonisamide measured by in vitro dissolution studies is reduced.

There is a need to improve on the preparation of pharmaceutical capsules containing zonisamide as the active ingredient. The present invention overcomes the problems encountered by prior art zonisamide capsules by providing novel stable zonisamide capsules prepared by a method exemplified by a novel method for preparing zonisamide pharmaceutical formulations. The zonisamide capsules of the invention prepared by the novel methods or similar method are stable in that no lumps are formed with the ingredients inside the capsules upon storage, resulting in stable dissolution rates.

### SUMMARY OF THE INVENTION

The present invention is directed toward a novel stable zonisamide pharmaceutical composition comprising a capsule shell filled with the intimate mixture of claim 1. The invention also provides the process of claim 10 for preparing the novel stable zonisamide pharmaceutical capsule, which process comprises:
(i) forming an intimate mixture of components by compressing, co-milling, co-micronizing and/or co-compacting the components, and/or by subjecting the components to one or more similarly intensive processes; and
(ii) filling capsule shells with the intimate mixture to obtain a stable zonisamide pharmaceutical capsule.

Alternatively, in the method for preparing the stable zonisamide pharmaceutical composition via the intimate mixture, one or more pharmaceutical excipients are mixed with the intimate mixture formed in step (i) to obtain a combination and then the capsule shells are filled with the combination in step (ii), wherein the "one or more pharmaceutical excipients" are one or more pharmaceutical excipients the same as or different from the at least one pharmaceutical excipient used in step (i) to form the intimate mixture. For instance, the "one or more pharmaceutical excipients" can be the pharmaceutically acceptable wetting agent used in the intimate mixture, or pharmaceutical acceptable excipients other than the pharmaceutically acceptable wetting agent.

The present invention includes the stable zonisamide pharmaceutical composition which can be in the form of the stable zonisamide pharmaceutical capsule prepared by the method described in the present patent application.

### DETAILED DESCRIPTION OF THE INVENTION

In the present patent application, when a pharmaceutical composition or formulation is described as stable, the term 'stable" means that the pharmaceutical composition or formulation has a dissolution rate that does not decrease, when subjected to accelerated storage conditions of 40° C at 75% relative humidity for 1, 2 and 3 months, by more than 10%, preferably by more than 5%, of the initial dissolution rate. Preferably, the solid ingredients in the "stable" pharmaceutical composition or formulation of the invention do not form lumps when subjected to accelerated storage conditions of 40° C at 75% relative humidity for 1, 2 and 3 months.

In the present invention dissolution rate may be measured using the following methodology: USP apparatus II (paddle), 75 rpm, 900 ml water at 37° C, sampling time 45 minutes and analysis with a UV detector at 284 nm.

Whether a pharmaceutical composition or formulation of the present invention forms lumps upon storage at accelerated conditions may be determined visually or via sieving analysis. If the composition or formulation is subjected to sieving analysis, the following procedure is performed. First, the smallest screen-mesh at least 99.5% of the composition or formulation is able to pass through is determined with a sample of the composition or formulation before storage at 40°C and 75% relative humidity. The smallest screen-mesh is hereinafter referred to as the "initial minimal screen-mesh." Another sample of the composition or formulation previously stored at 40°C and 75% relative humidity for 1, 2 or 3 months is then sieved with the initial minimal screen-mesh. If more than 97%, preferably more than 99%, of the post-storage sample is able to pass through the initial minimal screen-mesh, the composition or formulation is described as being lump free.

In the method for preparing the stable zonisamide pharmaceutical composition via the intimate mixture, the effective amount of the at least one pharmaceutically acceptable wetting agent in the intimate mixture in step (i) can be 0.1 wt% to 10 wt% the effective amount of the at least one pharmaceutically acceptable glidant is 0.1 wt% to 10 wt %, when the at least one pharmaceutically acceptable excipient includes one or more glidants, and 1 wt% to 90 wt% of all carbohydrates combined when the at least one pharmaceutically acceptable excipient includes one or more carbohydrates, wherein the wt% is based on the total weight of the intimate mixture. For instance, when the at least one pharmaceutical acceptable excipient in the intimate mixture comprises a pharmaceutically acceptable wetting agent, pharmaceutically acceptable glidant and pharmaceutically acceptable carbohydrate, the intimate mixture can contain, for instance, in addition to the zonisamide powder, 0.1 wt% to 10 wt% of the wetting agent, 0.1 wt% to 10 wt% of the glidant and 1 wt% to 90 wt% of the carbohydrate.

The pharmaceutically acceptable wetting agent can be selected from alkyl sulfates, e.g. sodium lauryl sulfate, sodium stearyl sulfate, sodium oleyl sulfate and sodium cetyl sulfate, alkyl aryl sulfonates, e.g. sodium dodecylbenzene sulfonate and dialkyl sodium sulfosuccinates, e.g. sodium bis-(2-ethylhexyl)sulfosuccinate, and most preferably sodium lauryl sulfate. Further examples of the pharmaceutically acceptable wetting agent include benzethonium chloride, cetylpyridinium chloride, docusate sodium, poloxamer, polysorbate and sorbitan esters. The pharmaceutically acceptable glidant can be selected from talc, calcium stearate, calcium phosphate tribasic, calcium silicate, powdered cellulose, magnesium silicate, magnesium trisilicate, starch and, preferably, colloidal silicon dioxide. The pharmaceutically acceptable carbohydrate can be selected from sucrose, mannitol, sorbitol, lactose and glucose, in solid form.

The stable zonisamide pharmaceutical composition in the form of a capsule prepared by the method described in the present patent application can contain 25, 50 or 100 mg zonisamide per capsule.

One of the objects of the present invention is to provide a solid pharmaceutical formulation, e.g. in the form of capsules, which releases from 60% to 100% of zonisamide as the active ingredient in 45 minutes at release in an aqueous environment. The release rate of the pharmaceutical formulation is maintained during the shelf life.

Another object of the present invention is drawn to a solid dosage form of zonisamide comprising the intimate mixture of claim 1, which intimate mixture comprises solid zonisamide powder and at least one pharmaceutically acceptable wetting agent, wherein the solid dosage form is stable such that the dissolution rate measured at a sampling time of 45 minutes, of the solid dosage form does not decrease by more than 10% than its initial dissolution rate upon storage at 40° C and 75% relative humidity for up to three months, when tested by the parameters described herein. Preferably, the solid dosage form is also stable in that no lumps are formed upon storage at 40° C and 75% relative humidity for up to three months.

In the present patent application, the term "intimate mixture" means a mixture more intimate than normal powder mixes, e.g., powder mixes prepared by dry mixing or blending of zonisamide powder with one or more pharmaceutical acceptable excipients. The term "intimate mixture" refers to a mixture of closely-packed components. The intimate mixture may be produced by co-milling, co-micronization and/or co-compaction of the components and similarly intensive processes.

Without being bound by any theory on how the present invention works, it is believed that the intimate mixture can render the zonisamide pharmaceutical composition or formulation stable because the at least one pharmaceutically acceptable excipient in the intimate mixture prevents the zonisamide powder to adhere to each other to form lumps over time with the at least one pharmaceutically acceptable excipient coating the zonisamide powder and separating the zonisamide powder from each other. By preventing the formation of lumps over time upon storage, the dissolution rate of the zonisamide pharmaceutical composition or formulation made from the intimate mixture can be maintained at a value that does not change more than 10% over time upon storage even for up to three months at accelerated conditions.

Some of the embodiments of the present invention are illustrated with the working examples below, which are for demonstration purposes. One skilled in the art can practice the invention beyond the scope of the working examples based on the disclosures in the present patent application.

### EXAMPLES

### Comparative Example 1-3

The following batches of a pharmaceutical composition containing zonisamide were prepared by a dry mix method. The ingredients of Part I were blended for 15 minutes to form an initial blend. Part II ingredients were added to the initial blend and the final blend was encapsulated into capsules. The Part I ingredients and Part II ingredients used are shown in Table 1.

**Table 1**

| Ingredient | Comp. Ex. 1 *K-33094** (mg/capsule) | Comp. Ex. 2 *K-33395** (mg/capsule) | Comp. Ex. 3 *K-33396** (mg/capsule) |
|---|---|---|---|
| Part I: | | | |
| Zonisamide | 100.0 | 100.0 | 100.0 |
| Microcrystalline cellulose | 170.0 | 170.0 | 140.0 |
| Colloidal silicon dioxide | 1.5 | 1.5 | 1.5 |
| Sodium lauryl sulfate | 1.5 | 1.5 | 1.5 |
| | | | |

| Part II: | | | |
|---|---|---|---|
| Microcrystalline cellulose | 55.5 | 55.5 | 45.5 |
| Colloidal silicon dioxide | 1.5 | 1.5 | 1.5 |
| Hydrogenated vegetable oil | 10.0 | 10.0 | 10.0 |
| | | | |
| Total | 340.0 | 340.0 | 300.0 |
| Capsule Shell No. | No.1 | No.0 | No.0 |

| | | | |
|---|---|---|---|
| *Batch number | | | |

### Working Example 1

The ingredients of Part I of Table 2 were granulated using a purified water as a granulating liquid. The granulate was dried, milled and blended with a Part II ingredient. The final blend was encapsulated into capsules. The ingredients of Part I and Part II used are shown in Table 2.

**Table 2**

| Ingredient | *K-34327** (mg/capsule) |
|---|---|
| Part I: | |
| Zonisamide | 207.9 |
| Microcrystalline cellulose | 100.0 |
| Sodium lauryl sulfate | 1.5 |
| Crospovidone | 20.0 |
| Povidone | 5.0 |
| | |

| Part II: | |
|---|---|
| Hydrogenated vegetable oil | 5.6 |
| | |
| Total | 340.0 |
| Capsule Shell No. | No.0 |

| | |
|---|---|
| *Batch number | |

### Working Example 2

Preparation of zonisamide capsule by dry mixing using an intimate admixture -

Part I materials were milled through 0.8 mm Frewitt oscillating granulator, blended with Part II materials and encapsulated into capsules. The materials of Parts I and II used are shown in Table 3.

**Table 3**

| Ingredient | *K-34393** ( mg/capsule) |
|---|---|
| Part I: | |
| Zonisamide | 100.0 |
| Colloidal silicon dioxide | 3.0 |
| Sodium lauryl sulfate | 3.0 |
| | |

| Part II: | |
|---|---|
| Microcrystalline cellulose | 224.0 |
| Hydrogenated vegetable oil | 10.0 |
| | |
| Total | 340.0 |
| Capsule Shell No. | No.0 |

| | |
|---|---|
| *Batch number | |

### Properties of Zonisamide Capsules of Comparative Examples and Working Examples

A stability test was performed on samples of Comparative Examples 1-3 (each batch in Table 1) initially after they were prepared and at various times after storage at accelerated conditions, i.e., 40° C and 75% relative humidity, for 1 or 2 months. The results are shown in Table 4.

Dissolution method: USP apparatus II (paddle), 75 rpm, 900 ml water at 37° C, sampling time 45 minutes, analysis using UV detector at 284 nm.

**Table 4.**

| Batch No. | Initial | 1 mon. 40°C/75% RH | 2 mon. 40°C/75% RH |
|---|---|---|---|
| *K-33094* | Avg. 94% | Avg. 74% | Avg.91 % |
| | Mini.* 88% | Mini.* 45% | Mini.* 80% |
| | | | |
| *K-33395* | | Avg. 80% | Avg. 76% |
| | | Mini.* 55% | Mini.* 51% |
| | | | |
| *K-33396* | | Avg. 91 % | Avg. 86% |
| | | Mini.* 87% | Mini.* 60% |

| | | | |
|---|---|---|---|
| *Minimum | | | |

The stability results generated on the zonisamide capsules of Comparative Examples 1-3 demonstrated reduction of the dissolution rates upon storage at accelerated conditions. Visual inspections of the zonisamide capsules of Comparative Examples 1-3 indicated that these capsules contained lumps of ingredients.

The water content of the zonisamide capsule of Comparative Example 1 was determined by the Karl Fischer method. The Karl Fischer results showed that the water content of the zonisamide capsule was maintained at the same level during storage at 40° C and 75% relative humidity, or 25° C and 60% relative humidity (Table 5).

**Table 5**

| Karl Fischer Results | | | | | |
|---|---|---|---|---|---|
| Batch No. | Initial | 1 mon., 40°C, 75% RH | 2 mon., 40°C, 75% RH | 3 mon., 40°C, 75% RH | 3 mon., 25°C, 60% RH |
| K-33094 | 3.4 | 3.6% | 4.0% | 4.0% | 3.7% |

A stability test was performed on samples of Working Examples 1 and 2 (each batch in Tables 2 and 3) initially after they were prepared and at various times after storage at accelerated conditions, i.e., 40° C and 75% relative humidity, for 1, 2 or 3 months. The results are shown in Table 6.

Dissolution method: USP apparatus II (paddle), 75 rpm, 900ml water at 37° C, sampling time 45 minutes, analysis using UV detector at 284 nm.

**Table 6.**

| Batch No. | Initial | 1 mon. 40° C 75% RH | 2 mon. 40° C 75% RH | 3 mon. 40° C 75% RH |
|---|---|---|---|---|
| *K-34327* | Avg.95% | Avg.88% | Avg.94% | Avg. 96% |
| | Mini.* 90% | Mini.* 78% | Mini.* 91% | Mini.* 92% |
| | | | | |
| *K-34393* | Avg. 93% | Avg. 96% | Avg. 97% | Avg. 94% |
| | Mini.* 90% | Mini.* 93% | Mini.* 95% | Mini.* 90% |

| | | | | |
|---|---|---|---|---|
| *Minimum | | | | |

Visual inspections of the zonisamide capsules of Working Examples 1 and 2 did not show any lumps. The zonisamide capsules also proved to be stable. The dissolution results demonstrated that the zonisamide capsules of Working Examples 1 (K-34327) and 2 (K-34393) were stable.

## Claims

1. A stable pharmaceutical dosage form comprising, in intimate mixture, solid particulate zonisamide and at least one pharmaceutically acceptable wetting agent selected from alkyl sulfates, alkyl aryl sulfonates, dialkyl sodium sulfosuccinates, benzethonium chloride, cetylpyridinium chloride, docusate sodium, poloxamer, polysorbate and sorbitan esters.

2. The stable pharmaceutical dosage form of claim 1, wherein the at least one pharmaceutically acceptable wetting agent is selected from the group consisting of sodium lauryl sulfate, sodium stearyl sulfate, sodium oleyl sulfate, sodium cetyl sulfate, sodium dodecylbenzene sulfonate, sodium bis-(2-ethylhexyl)sulfosuccinate, benzethonium chloride, cetylpyridinium chloride, docusate sodium, poloxamer, polysorbate and sorbitan esters.

3. The stable pharmaceutical dosage form of claim 2, wherein the at least one pharmaceutically acceptable wetting agent comprises sodium lauryl sulfate.

4. The stable pharmaceutical dosage form of any preceding claim, comprising at least one further pharmaceutically acceptable excipient selected from pharmaceutically acceptable glidants, pharmaceutically acceptable carbohydrates and combination of two or more thereof.

5. The stable pharmaceutical dosage form of claim 4 wherein the at least one pharmaceutically acceptable glidant is selected from talc, calcium stearate, calcium phosphate tribasic, calcium silicate, powdered cellulose, magnesium silicate, magnesium trisilicate, starch and colloidal silicon dioxide.

6. The stable pharmaceutical dosage form of claim 5, wherein the at least one pharmaceutically acceptable glidant comprises colloidal silicon dioxide.

7. The stable pharmaceutical dosage form of claim 4, wherein the at least one pharmaceutically acceptable carbohydrate is selected from sucrose, glucose, lactose, mannitol and sorbitol.

8. The stable pharmaceutical dosage form of any preceding claim, wherein no lumps are formed in the dosage form upon storage at 40° C and 75% relative humidity for up to three months.

9. The stable pharmaceutical dosage form of any preceding claim, wherein the dissolution rate of the zonisamide from the solid dosage form, as measured using the USP apparatus II (paddle), 75 rpm, 900ml water at 37°C over 45 minutes using a UV detector at 284 nm, does not decrease by more than 10%, preferably by not more than 5%, from its initial dissolution rate upon storage at 40° C and 75% relative humidity for up to three months.

10. A process for preparing a stable zonisamide pharmaceutical capsule, comprising
(i) forming an intimate mixture of components, wherein the components comprise zonisamide powder and an effective amount of at least one pharmaceutically acceptable excipient comprising a wetting agent selected from alkyl sulfates, alkyl aryl sulfonates, dialkyl sodium sulfosuccinates, benzethonium chloride, cetylpyridinium chloride, docusate sodium, poloxamer, polysorbate and sorbitan esters; and
(ii) filling capsule shells with the intimate mixture to obtain the stable zonisamide pharmaceutical capsule.

11. The process of claim 10, wherein the at least one pharmaceutically acceptable wetting agent is selected from the group consisting of sodium lauryl sulfate, sodium stearyl sulfate, sodium oleyl sulfate, sodium cetyl sulfate, sodium dodecylbenzene sulfonate, sodium bis-(2-ethylhexyl)sulfosuccinate, benzethonium chloride, cetylpyridinium chloride, docusate sodium, poloxamer, polysorbate and sorbitan esters.

12. The process of claim 11, wherein the at least one pharmaceutically acceptable wetting agent comprises sodium lauryl sulfate.

13. The process of any of claims 10 to 12, comprising at least one further pharmaceutically acceptable excipient in step (i) selected from pharmaceutically acceptable carbohydrates, pharmaceutically acceptable glidants and combinations of two or more thereof.

14. The process of any of claims 10 to 13, wherein the effective amount of the at least one pharmaceutically acceptable wetting agent is 0.1 wt% to 10 wt%, the effective amount of the at least one pharmaceutically acceptable glidant is 0.1 wt% to 10 wt%, when the at least one pharmaceutically acceptable excipient includes one or more glidants, and I wt% to 90 wt% of one or more carbohydrates combined when the at least one pharmaceutically acceptable excipient includes one or more carbohydrates, wherein the wt% is based on the total weight of the intimate mixture.

15. The process of claim 13 or claim 14, wherein the at least one pharmaceutically acceptable glidant is selected from talc, calcium stearate, calcium phosphate tribasic, calcium silicate, powdered cellulose, magnesium silicate, magnesium trisilicate, starch and colloidal silicon dioxide.

16. The process of claim 15, wherein the at least one pharmaceutically acceptable glidant comprises colloidal silicon dioxide.

17. The process of any of claims 10 to 16, wherein one or more additional pharmaceutical acceptable excipients are mixed with the intimate mixture between step (i) and step (ii) to obtain a combination, wherein in step (ii) the capsule shells are filled with the combination to form the capsule.

18. The process of claim 17, wherein the one or more additional pharmaceutical acceptable excipients are one or more pharmaceutical excipients other than the at least one pharmaceutically acceptable excipient included in the intimate mixture of step (i).

19. The process of claim 18, wherein the one or more pharmaceutical acceptable excipients are selected from pharmaceutically acceptable diluents, binders and disintegrants.

20. The process of any of claims 10 to 19, wherein the intimate mixture is formed by compressing, co-milling, co-micronizing and/or co-compacting the components.

21. A stable zonisamide pharmaceutical composition prepared by the process of any of claims 10 to 20.

## Patentansprüche

1. Stabile pharmazeutische Dosierungsform, die folgendes umfasst: ein inniges Gemisch aus festem, teilchenförmigen Zonisamid und wenigstens ein pharmazeutisch verträgliches Benetzungsmittel, ausgewählt unter Alkylsulfaten, Alkylarylsulfonaten, Dialkylnatriumsulfosuccinaten, Benzethoniumchlorid, Cetylpyridiniumchlorid, Docusat-Natrium, Poloxamer, Polysorbat und Sorbitanestern.

2. Stabile pharmazeutische Dosierungsform nach Anspruch 1, wobei das wenigstens eine pharmazeutisch verträgliche Benetzungsmittel ausgewählt ist aus der Gruppe, bestehend aus Natriumlaurylsulfat, Natriumstearylsulfat, Natriumoleylsulfat, Natriumcetylsulfat, Natriumdodecylbenzolsulfonat, Natrium-bis-(2-ethyl-hexyl)sulfosuccinat, Benzethoniumchlorid, Cetylpyridiniumchlorid, Docusat-Natrium, Poloxamer, Polysorbat und Sorbitanestern.

3. Stabile pharmazeutische Dosierungsform gemäß Anspruch 2, wobei das wenigstens eine pharmazeutisch verträgliche Benetzungsmittel Natriumlaurylsulfat umfasst.

4. Stabile pharmazeutische Dosierungsform gemäß einem der vorangehenden Ansprüche, die wenigstens ein weiteres pharmazeutisch verträgliches Bindemittel aufweist, das unter pharmazeutisch verträglichen Fließregulierungsmitteln, pharmazeutisch verträglichen Kohlenhydraten und Kombinationen von zwei oder mehr davon ausgewählt ist.

5. Stabile pharmazeutische Dosierungsform nach Anspruch 4, wobei das wenigstens eine pharmazeutisch verträgliche Fließregulierungsmittel ausgewählt ist unter Talk, Kalziumstearat, dreibasischem Kalziumphosphat, Kalziumsilicat, pulverförmiger Cellulose, Magnesiumsilicat, Magnesiumtrisilicat, Stärke und kolloidalem Siliziumdioxid.

6. Stabile pharmazeutische Dosierungsform nach Anspruch 5, wobei das wenigstens eine pharmazeutisch verträgliche Fließregulierungsmittel kolloidales Siliziumdioxid umfasst.

7. Stabile pharmazeutische Dosierungsform nach Anspruch 4, wobei das wenigstens eine pharmazeutisch verträgliche Kohlenhydrat ausgewählt ist unter Saccharose, Glukose, Laktose, Mannitol und Sorbitol.

8. Stabile pharmazeutische Dosierungsform gemäß einem der vorangehenden Ansprüche, wobei nach einer Lagerung bei 40 °C und 75 % relativer Feuchte für bis zu drei Monate keine Klumpen in der Dosierungsform gebildet werden.

9. Stabile pharmazeutische Dosierungsform nach einem der vorangehenden Ansprüche, wobei die Auflösungsgeschwindigkeit des Zonisamids in der festen Dosierungsform nicht mehr als 10 %, bevorzugt nicht mehr als 5 %, der anfänglichen Auflösungsgeschwindigkeit abnimmt, nach Lagerung bei 40 °C und 75 % relativer Feuchte für bis zu drei Monate, gemessen unter Verwendung des USP Apparats II (Paddel), bei 75 Upm, 900 ml Wasser bei 37 °C während 45 Minuten, unter Verwendung eines UV-Detektors bei 284 nm.

10. Verfahren zur Herstellung einer stabilen pharmazeutischen Zonisamid-Kapsel, welches folgende Stufen umfasst:
(i) Bilden eines innigen Gemisches von Komponenten, wobei die Komponenten Zonisamidpulver und eine wirksame Menge wenigstens eines pharmazeutisch verträglichen Bindemittels umfassen, welches ein Benetzungsmittel umfasst, ausgewählt unter Alkylsulfaten, Alkylarylsulfonaten, Dialkylnatriumsulfosuccinaten, Benzethoniumchlorid, Cetylpyridiniumchlorid, Docusat-Natrium, Poloxamer, Polysorbat und Sorbitanestern, und
(ii) Befüllen von Kapselhüllen mit dem innigen Gemisch, unter Erhalt der stabilen pharmazeutischen Zonisamid-Kapsel,

11. Verfahren nach Anspruch 10, wobei das wenigstens eine pharmazeutisch verträgliche Benetzungsmittel ausgewählt ist aus der Gruppe, bestehend aus Natriumlaurylsulfat, Natriumstearylsulfat, Natriumoleylsulfat, Natriumcetylsulfat, Natriumdodecylbenzolsulfonat, Natrium-bis-(2-ethylhexyl)sulfosuccinat, Benzethoniumchlorid, Cetylpyridiniumchlorid, Docusat-Natrium, Poloxamer, Polysorbat und Sorbitanestern.

12. Verfahren nach Anspruch 11, wobei das pharmazeutisch verträgliche Benetzungsmittel Natriumlaurylsulfat umfasst.

13. Verfahren nach einem der Ansprüche 10 bis 12, welches wenigstens ein weiteres pharmazeutisch verträgliches Bindemittel in Stufe (i) umfasst, das ausgewählt ist unter pharmazeutisch verträglichen Kohlenhydraten, pharmazeutisch verträglichen Fließregulierungsmitteln und Kombinationen von zwei oder mehreren davon.

14. Verfahren nach einem der Ansprüche 10 bis 13, wobei die effektive Menge des wenigstens einen pharmazeutisch verträglichen Benetzungsmittels 0,1 Gew.-% bis 10 Gew.-% beträgt, die effektive Menge des wenigstens einen pharmazeutisch verträglichen Ffießreguüerungsmittels 0,1 Gew.-% bis 10 Gew.-% beträgt, wenn das wenigstens eine pharmazeutisch verträgliche Bindemittel ein oder mehrere Fließregulierungsmittel beinhaltet, und 1 Gew.-% bis 90 Gew.-% der zusammengenommenen einen oder mehreren Kohlenhydrate, wenn das wenigstens eine pharmazeutisch verträgliche Bindemittel ein oder mehrere Kohlenhydrate einschließt, wobei Gew.-% auf dem Gesamtgewicht des innigen Gemisches basiert.

15. Verfahren gemäß Anspruch 13 oder 14, wobei das wenigstens eine pharmazeutisch verträgliche Fließregulierungsmittel ausgewählt ist unter Talk, Kalziumstearat, tribasischem Kalziumphosphat, Kalziumsilicat, pulverförmiger Cellulose, Magnesiumsilicat, Magnesiumtrisilicat, Stärke und kolloidalem Siliziumdioxid.

16. Verfahren gemäß Anspruch 15, wobei das wenigstens eine pharmazeutisch verträgliche Fließregulierungsmittel kolloidales Siliziumdioxid umfasst.

17. Verfahren nach einem der Ansprüche 10 bis 16, wobei ein oder mehrere zusätzliche pharmazeutisch verträgliche Bindemittel mit dem innigen Gemisch zwischen Stufe (i) und Stufe (ii) gemischt werden, unter Erhalt einer Kombination, wobei in Stufe (ii) die Kapselhüllen mit der Kombination unter Bildung der Kapsel gefüllt werden.

18. Verfahren gemäß Anspruch 17, wobei die einen oder mehreren zusätzlichen pharmazeutisch verträglichen Bindemittel ein oder mehrere pharmazeutische Bindemittel sind, die sich von dem wenigstens einen pharmazeutisch verträglichen Bindemittel, das in dem innigen Gemisch von Stufe (i) enthalten ist, unterscheiden.

19. Verfahren gemäß Anspruch 18, wobei das eine oder mehrere pharmazeutisch veträgliche Bindemittel ausgewählt sind unter pharmazeutisch verträglichen Verdünnungsmitteln, Bindern und Sprengmitteln.

20. Verfahren gemäß einem der Ansprüche 10 bis 19, wobei das innige Gemisch durch Komprimieren, gemeinsam Mahlen, gemeinsam Mikronisieren und/oder gemeinsam Verdichten der Komponenten gebildet wird.

21. Stabile pharmazeutische Zonisamid-Zusammensetzung, die durch das Verfahren nach einem der Ansprüche 10 bis 20 hergestellt wird.

## Revendications

1. Forme pharmaceutique stable comprenant, en mélange intime, du zonisamide en particules solides et au moins un agent de mouillage pharmaceutiquement acceptable choisi parmi des sulfates d'alkyle, des sulfonates d'alkylaryle, des sulfosuccinates de dialkyl-sodium, le chlorure de benzéthonium, le chlorure de cétyl-pyridinium, le docusate sodique, un poloxamère, un polysorbate et des esters de sorbitan.

2. Forme pharmaceutique stable selon la revendication 1, dans laquelle au moins un agent de mouillage pharmaceutiquement acceptable est choisi dans le groupe constitué de laurylsulfate de sodium, stéarylsulfate de sodium, oléylsulfate de sodium, cétylsulfate de sodium, docécylbenzène-sulfonate de sodium, bis-(2-éthylhexyl)-sulfosuccinate de sodium, chlorure de benzéthonium, chlorure de cétyl-pyridinium, docusate sodique, un poloxamère, un polysorbate et des esters de sorbitan.

3. Forme pharmaceutique stable selon la revendication 2, dans laquelle au moins un agent de mouillage pharmaceutiquement acceptable comprend du laurylsulfate de sodium.

4. Forme pharmaceutique stable selon l'une quelconque des revendications précédentes, comprenant au moins un autre excipient pharmaceutiquement stable choisi parmi des glissants pharmaceutiquement stables, des glucides pharmaceutiquement stables et une combinaison de deux ou plus de ceux-ci.

5. Forme pharmaceutique stable selon la revendication 4, dans laquelle le au moins un glissant pharmaceutiquement acceptable est choisi parmi le talc, le stéarate de calcium, le phosphate de calcium tribasique, le silicate de calcium, la cellulose en poudre, le silicate de magnésium, le trisilicate de magnésium, l'amidon et la silice colloïdale.

6. Forme pharmaceutique stable selon la revendication 5, dans laquelle le au moins un glissant pharmaceutiquement acceptable comprend de la silice colloïdale.

7. Forme pharmaceutique stable selon la revendication 4, dans laquelle le au moins un glucide pharmaceutiquement acceptable est choisi parmi le saccharose, le glucose, le lactose, le mannitol et le sorbitol.

8. Forme pharmaceutique stable selon l'une quelconque des revendications précédentes, dans laquelle aucun grumeau ne s'est formé dans la forme pharmaceutique lors du stockage à 40° C et 75 % d'humidité relative jusqu'à trois mois.

9. Forme pharmaceutique stable selon l'une quelconque des revendications précédentes, dans laquelle le taux de dissolution du zonisamide à partir de la forme pharmaceutique solide, mesurée en utilisant l'appareil II (à palette) de la Pharmacopée américaine, 75 tr/min, 900 ml d'eau à 37° C sur 45 minutes en utilisant un détecteur UV à 284 nm, ne diminue pas de plus de 10 %, de préférence de pas plus de 5 %, à partir du taux de dissolution initial après stockage à 40° C et 75 % d'humidité relative jusqu'à trois mois.

10. Procédé de préparation d'une capsule pharmaceutique de zonisamide stable, comprenant
(i) la formation d'un mélange intime des composants, où les composants comprennent du zonisamide en poudre et une quantité efficace d'au moins un excipient pharmaceutiquement acceptable comprenant un agent de mouillage choisi parmi des sulfates d'alkyle, des sulfonates d'alkylaryle, des sulfosuccinates de dialkyl-sodium, le chlorure de benzéthonium, le chlorure de cétyl-pyridinium, le docusate sodique, un poloxamère, un polysorbate et des esters de sorbitan ; et
(ii) le remplissage des enveloppes de capsules avec le mélange intime pour obtenir la capsule pharmaceutique de zonisamide stable.

11. Procédé selon la revendication 10, dans laquelle le au moins un agent de mouillage pharmaceutiquement acceptable est choisi dans le groupe constitué de laurylsulfate de sodium, stéarylsulfate de sodium, oléylsulfate de sodium, cétylsulfate de sodium, docécylbenzène-sulfonate de sodium, bis-(2-éthylhexyl)-sulfosuccinate de sodium, chlorure de benzéthonium, chlorure de cétyl-pyridinium, docusate sodique, un poloxamère, un polysorbate et des esters de sorbitan.

12. Procédé selon la revendication 11, dans lequel le au moins un agent de mouillage pharmaceutiquement acceptable comprend du laurylsulfate de sodium.

13. Procédé selon l'une quelconque des revendications 10 à 12, comprenant au moins un autre excipient pharmaceutiquement acceptable dans l'étape (i) choisi parmi des glucides pharmaceutiquement acceptables, des glissants pharmaceutiquement acceptables et des combinaisons de deux ou plus de ceux-ci.

14. Procédé selon l'une quelconque des revendications 10 à 13, dans lequel la quantité efficace du au moins un agent de mouillage pharmaceutiquement acceptable est de 0,1 % en poids à 10 % en poids, la quantité efficace du au moins un glissant pharmaceutiquement acceptable est de 0,1 % en poids à 10 % en poids, lorsque le au moins un excipient pharmaceutiquement acceptable comprend un ou plusieurs glissants et 1 % en poids à 90 % en poids d'un ou plusieurs glucides combinés lorsque le au moins un excipient pharmaceutiquement acceptable comprend un ou plusieurs glucides, où le pourcentage en poids est basé sur le poids total du mélange intime.

15. Procédé selon la revendication 13 ou la revendication 14, dans lequel le au moins un glissant pharmaceutiquement acceptable est choisi parmi le talc, le stéarate de calcium, le phosphate de calcium tribasique, le silicate de calcium, la cellulose en poudre, le silicate de magnésium, le trisilicate de magnésium, l'amidon et la silice colloïdale.

16. Procédé selon la revendication 15, dans lequel le au moins un glissant pharmaceutiquement acceptable comprend de la silice colloïdale.

17. Procédé selon l'une quelconque des revendications 10 à 16, dans lequel un ou plusieurs excipients pharmaceutiquement acceptables supplémentaires sont mélangés avec le mélange intime entre l'étape (i) et l'étape (ii) pour obtenir une combinaison, où dans l'étape (ii) les enveloppes de capsules sont remplies avec la combinaison pour former la capsule.

18. Procédé selon la revendication 17, dans lequel le(s) un ou plusieurs excipients pharmaceutiquement acceptables supplémentaires sont un ou plusieurs excipients pharmaceutiques autres que le au moins un excipient pharmaceutiquement acceptable inclus dans le mélange intime de l'étape (i).

19. Procédé selon la revendication 18, dans lequel le(s) un ou plusieurs excipients pharmaceutiques acceptables sont choisis parmi des diluants, des agents de liaison et des délitants pharmaceutiquement acceptables.

20. Procédé selon l'une quelconque des revendications 10 à 19, dans lequel le mélange intime est formé par compression, cobroyage, comicronisation et/ou cocompactage des composants.

21. Composition pharmaceutique de zonisamide stable préparée par le procédé selon l'une quelconque des revendications 10 à 20.
